# FASCICULE DE BREVET EUROPEEN

(11) **EP 2 320 837 B1**
(45) Date de publication et mention de la délivrance du brevet: **22.02.2012**
(21) Numéro de dépôt: 09772656.6
(22) Date de dépôt: 10.06.2009
(51) Int. Cl.: A61F 5/01

(54) **MECANISME D'EPAULE POUR ORTHESE**
SCHULTERMECHANISMUS FÜR EINE ORTHESE
SHOULDER MECHANISM FOR AN ORTHOSIS

(30) Priorité: 10.06.2008 FR 0803204
(43) Date de publication de la demande: 18.05.2011
(73) Titulaire: Commissariat à l'Énergie Atomique et aux Énergies Alternatives, 75015 Paris (FR)
(72) Inventeur: GARREC, Philippe, F-91190 Gif Sur Yvette (FR); MARTINS, Jean-Pierre, F-84530 Villelaure (FR)
(74) Mandataire: Parzy, Benjamin Alain
(86) Numéro de dépôt international: PCT/FR2009/000691
(87) Numéro de publication internationale: WO 2010/000973

(56) Documents cités:
- WO-A-2008/028190
- US-A- 4 651 719
- US-A- 5 407 420
- US-A- 5 538 499
- US-A1- 2004 153 010

## Description

L'invention concerne un mécanisme d'épaule pour orthèse.

### ARRIERE-PLAN DE L'INVENTION

Une telle orthèse comporte en général un bras au bout duquel un avant-bras est articulé. Le bras est articulé par une articulation d'épaule à un support. Le support peut être immobile, ou au contraire être porté par l'utilisateur de l'orthèse. Il s'étend en général derrière l'utilisateur, pour éviter toute interférence lors de la manipulation. Ce support est dès lors appelé dossier.

L'articulation d'épaule peut en pratique être approchée par une liaison rotule, ce dans une large plage de débattement. Il est connu de réaliser une telle liaison par l'utilisation de trois liaisons tournantes d'axes concourants.

A cet effet, un premier élément intermédiaire est monté pour tourner sur le support selon un premier axe d'articulation. Un deuxième élément intermédiaire est monté pour tourner sur le premier élément intermédiaire selon un deuxième axe d'articulation perpendiculaire au premier et concourant avec celui-ci. Enfin, le bras de l'orthèse est monté pour tourner sur le deuxième élément intermédiaire selon un troisième axe d'articulation perpendiculaire aux premier et deuxième axes et concourant avec ceux-ci. La disposition des axes d'articulation s'entend alors que le bras est au repos le long du corps. Quelque soit la position du bras, les trois axes d'articulation restent sensiblement concourants.

Diverses réalisations sont connues. Partant du support, la première liaison tournante peut comporter un axe d'articulation qui s'étend soit verticalement, soit horizontalement. Dans ce dernier cas, l'axe d'articulation peut s'étendre d'arrière en avant par référence à l'utilisateur, selon une direction que l'on appellera ici longitudinale, ou encore selon une direction transversale, définie par les deux épaules de l'utilisateur et perpendiculaire à la direction longitudinale. Les directions des axes sont ici données à titre indicatif, et peuvent présenter des orientations légèrement différentes, résultant par exemple d'une optimisation.

Des orthèses dont l'axe d'articulation de la première liaison tournante s'étend verticalement ont été proposées, mais elles présentent l'inconvénient que le mécanisme d'articulation qui matérialise cette première liaison tournante s'étend en saillie au dessus de l'épaule, donc au voisinage de la tête de l'utilisateur, ce qui est gênant pour l'utilisateur au moins parce que ce mécanisme se trouve en permanence dans son champ de vision. En outre, une telle disposition conduit à une interférence avec l'extrémité de l'avant-bras lorsque le manipulateur de l'orthèse replie l'avant-bras vers l'épaule.

Des orthèses dont l'axe de la première liaison tournante s'étend selon une direction horizontale transversale sont réalisables, mais le mécanisme qui matérialise cette liaison tournante s'étend alors en saillie sur le côté de l'épaule et est susceptible d'interférer avec l'orthèse lors de la manipulation de celle-ci.

L'arrière-plan technologique est notamment illustré par le document US 5 407 420 qui décrit un mécanisme d'épaule pour orthèse selon le préambule de la revendication 1.

L'invention a plus particulièrement trait à un mécanisme d'épaule dont l'axe de la première liaison tournante s'étend selon une direction longitudinale.

### OBJET DE L'INVENTION

L'invention a pour objet un mécanisme d'épaule pour orthèse dont le mécanisme d'articulation d'épaule est conçu pour minimiser à la fois le risque de singularité par alignement des axes des liaisons tournantes, et le risque d'interférence avec l'orthèse lors de la manipulation de celle-ci.

### BREVE DESCRIPTION DE L'INVENTION

En vue de la réalisation de ce but, on propose un mécanisme d'épaule pour orthèse, assurant une articulation de type rotulaire entre un support et un bras d'orthèse, comportant un premier élément intermédiaire monté pour tourner sur le support autour d'un premier axe d'articulation, un deuxième élément intermédiaire monté pour tourner sur le premier élément intermédiaire selon un deuxième axe d'articulation, le bras étant monté pour tourner sur le deuxième élément intermédiaire selon un troisième axe d'articulation, le premier axe d'articulation s'étendant selon une direction longitudinale, dans lequel selon l'invention le premier élément intermédiaire comporte un rail circulaire sur lequel le deuxième élément intermédiaire coulisse et qui présente un axe géométrique définissant le deuxième axe d'articulation qui s'étend verticalement lorsque le bras est au repos, le rail s'étendant pour faire extérieurement le tour de l'épaule.

Cette disposition offre plusieurs avantages. L'utilisation d'un premier axe qui s'étend selon une direction longitudinale permet de placer le mécanisme d'actionnement de la liaison tournante correspondante sur le support, derrière l'utilisateur, de sorte que ce mécanisme d'actionnement ne risque pas d'interférer avec le bras. Quant au rail circulaire, il permet une articulation selon un axe vertical, mais il s'étend sensiblement horizontalement de sorte qu'il ne gêne pas la vision de l'utilisateur et ne risque par d'interférer avec l'extrémité de l'avant-bras lorsque celui-ci est ramené vers l'épaule. Seule une situation dans laquelle le bras serait ramené latéralement à l'horizontale ferait basculer le rail autour du premier axe d'articulation d'une position sensiblement horizontale à une position sensiblement verticale, venant alors interférer avec le champ de vision de l'utilisateur. Mais cette situation est rare en pratique. Enfin, le risque de singularité par alignement du troisième axe avec le premier axe est facilement éliminé en prévoyant une limitation de course le long du rail circulaire empêchant cet alignement.

### BREVE DESCRIPTION DES DESSINS

L'invention sera mieux comprise à la lumière des figures des dessins annexés, parmi lesquelles :
- la figure 1 est un schéma cinématique de principe illustrant les diverses liaisons tournantes d'un mécanisme d'épaule selon l'invention, l'utilisateur étant symboliquement représenté en pointillés et est vu ici de dos ;
- la figure 2 est un schéma montrant un principe d'actionnement du mécanisme d'épaule de la figure 1, selon un mode particulier de réalisation de l'invention.

### DESCRIPTION DETAILLEE DE L'INVENTION

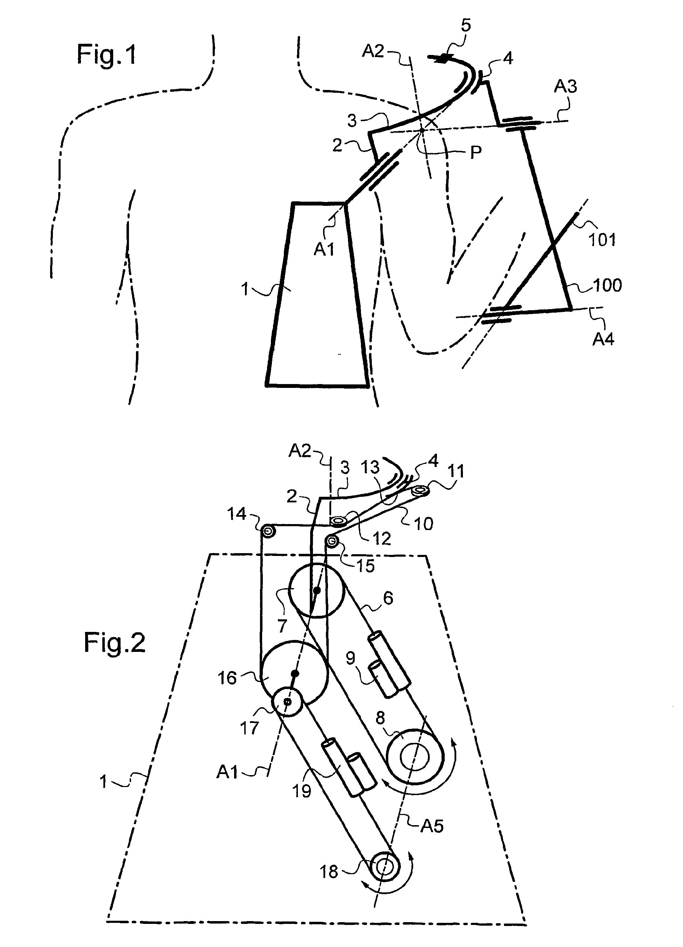

En référence à la figure 1, le mécanisme d'épaule de l'invention réalise une liaison de type rotulaire entre un support 1, ici un dossier contre lequel l'utilisateur s'adosse lors de la manipulation de l'orthèse, et un bras 100. Pour ce faire, le mécanisme d'épaule de l'invention comporte un premier élément intermédiaire 2 qui est monté tournant sur le support 1 au moyen d'un pivot d'axe A1 s'étendant selon une direction horizontale longitudinale (c'est-à-dire, pour rappel, d'arrière en avant en référence à l'utilisateur). Le premier élément intermédiaire 2 comporte un rail circulaire 3 s'étendant selon un arc de cercle centré sur un axe géométrique A2 qui s'étend verticalement et qui est concourant avec l'axe A1. Ici, le premier élément intermédiaire 2 est représenté dans la position qui correspond à la position de repos du bras le long du corps de l'utilisateur. Dans cette position, le rail circulaire 3 fait le tour de l'épaule, extérieurement à celle-ci, et il s'étend sensiblement dans un plan horizontal. Le champ de vision de l'utilisateur est complètement dégagé lorsqu'il regarde du côté de l'orthèse.

Sur le rail circulaire 3 du premier élément intermédiaire coulisse un chariot formant un deuxième élément intermédiaire 4 qui se déplace donc selon un arc de cercle centré sur l'axe A2. Ainsi, le deuxième élément intermédiaire 4 est-il relié au premier élément intermédiaire par une liaison tournante d'axe A2.

Enfin, le bras 100 est monté tournant sur le deuxième élément intermédiaire 4 par un pivot d'axe A3 s'étendant selon une direction transversale. L'axe A3 est perpendiculaire aux axes A1 et A2, et est concourant avec ceux-ci au point P de la figure coïncidant sensiblement avec l'épaule de l'opérateur.

Les trois liaisons tournantes ainsi organisées, dont les axes restent concourants quelque soit la position du bras forment ensemble la liaison rotulaire entre le support 1 et le bras 100.

Une butée de fin de course 5 disposée sur le rail circulaire 3 empêche le deuxième élément intermédiaire 4 de venir dans une position pour laquelle l'axe A3 serait aligné avec l'axe A1, évitant ainsi de façon très simple une singularité d'alignement.

Le bras 100 porte un avant-bras 101 qui est articulé sur le bras 100 selon un axe A4 parallèle à l'axe A3.

Selon un aspect particulier de l'invention illustré à la figure 2, la rotation du premier élément intermédiaire 2 autour de l'axe A1 est ici commandée à l'aide d'une transmission à câble 6 qui s'étend entre une première poulie 7 attelée au premier élément intermédiaire 2 et une deuxième poulie 8 d'axe parallèle A5 montée pivotante sur le support 1. Un actionneur 9 agit sur le câble pour le faire avancer ou reculer, et ainsi provoquer une rotation de la première poulie 7, qui provoque à son tour une rotation du premier élément intermédiaire, entraînant le bras dans une rotation autour de l'axe A1.

Selon un autre aspect particulier de l'invention, le coulissement du deuxième élément intermédiaire 4 le long du rail circulaire 3 est également commandé à l'aide d'une transmission à câble 10. Plus précisément, des poulies 11,12 montées pivotantes sur le premier élément intermédiaire 2 organisent un passage du câble 10 à proximité du rail circulaire 3. Le deuxième élément intermédiaire 4 est attelé au câble 10 en un point 13. Puis des poulies 14,15 montées également pivotantes sur le premier élément intermédiaire 2 organisent le renvoi du câble 10 vers une poulie menante 16 montée pivotante sur le support 1 selon l'axe A1. La poulie menante 16 est à son tour entraînée en rotation par un ensemble à câble (poulies 17,18, actionneur 19) en tout point semblable à celui qui sert à commander la rotation du premier élément intermédiaire 2 autour de l'axe A1, dans lequel la poulie 17 est attelée à la poulie menante 16 pour entraîner celle-ci en rotation, et ainsi provoquer un défilement du câble 10 qui provoque un coulissement du deuxième élément intermédiaire 4 le long du rail circulaire 3. Les poulies 7, 17 d'une part, et 8,18 d'autre part, sont coaxiales.

L'ensemble d'actionnement composé des poulies 7,8, de l'actionneur 9 et du câble associé d'une part, et l'ensemble d'actionnement composé des poulies 17,18, de l'actionneur 19 et du câble associé d'autre part, s'étendent sur le support 1 derrière l'utilisateur, de sorte que le champ de vision de l'utilisateur n'est pas altéré, cependant que tout risque d'interférence avec le bras est évité.

Quant à l'ensemble composé du câble 10 et des poulies 11,12,14,15, il est solidaire du premier élément intermédiaire 2 et suit donc celui-ci lors de ses rotations autour de l'axe A1. Il conviendra, pour éviter tout coulissement parasite du deuxième élément intermédiaire 3 le long du rail circulaire 2 lors d'une rotation du premier élément intermédiaire 2 autour de l'axe A1, d'organiser une rotation équivalente de la poulie menante 16 au moyen de l'ensemble d'actionnement correspondant.

Sur la figure 2, les arbres, liaisons et autres supports qui permettent de solidariser les poulies 11,12,14,15 avec le premier élément intermédiaire 2 et les arbres, liaisons et autres supports qui permettent de solidariser les poulies 7,8,18,17,18 et les actionneurs 9,19 au support 1 ne sont pas représentées, pour plus de clarté.

Ici, les axes A1,A2 et A3 sont concourants, de sorte que la liaison ainsi définie est une liaison rotulaire parfaite (aux incertitudes de positionnement et déformation près). Cependant, il est également possible de concevoir un mécanisme d'épaule pour orthèse selon l'invention dont les axes ne sont pas exactement concourants.

En réalité, les articulations d'épaules humaines ne correspondent pas à des liaisons rotulaires parfaites. En pratique, on peut définir, pour une articulation d'épaule à appareiller, une sphère de dispersion qui est la plus petite sphère dans laquelle se meut un point quelconque du bras de la personne appareillée lors des mouvements du bras dans l'espace. Cette sphère peut être déterminée par des procédés expérimentaux, notamment des mesures de débattement. Cette sphère est notamment caractérisée par un rayon appelé ici R_{humérus} et est centrée en un point proche d'un centre théorique d'une liaison rotulaire parfaite qui approcherait au mieux l'articulation en question. Le rayon R_{humérus} de la sphère de dispersion d'une épaule humaine est typiquement de l'ordre de quelques centimètres.

Il sera alors avantageux de définir un mécanisme d'épaule d'ortrèse selon l'invention ayant une sphère de dispersion dont le centre coïncide avec celle de la sphère de dispersion de l'articulation à appareiller, et dont le rayon R_{orthèse} est au plus égal au rayon de la sphère de dispersion du bras R_{humérus}. Ici, le rayon R_{orthèse} de la sphère de dispersion du mécanisme d'épaule de l'invention, appelé aussi défaut de concourance, est par définition la somme de la distance de l'axe A1 à l'axe A2 et de la distance de l'axe A2 à l'axe A3. Les distances mentionnées correspondent à la longueur d'un segment perpendiculaire aux deux axes concernés.

Si le rayon R_{humérus} de la sphère de dispersion de l'épaule à appareiller est égal à 40 millimètres, alors on retiendra un défaut de concourance égal au plus à 40 millimètres, et on pourra alors concevoir un mécanisme d'épaule d'orthèse selon l'invention dont les axes sont par exemple espacés deux à deux d'une distance égale à 20 millimètres. Bien sûr, un mécanisme tel qu'illustré à la figure 1 a ses axes concourants, de sorte que le défaut de concourance est nul.

De préférence, le mécanisme d'épaule d'orthèse est pourvu de moyens de réglage pour faire coïncider le centre de la sphère de dispersion de l'épaule appareillée et le centre de la sphère de dispersion de l'orthèse ainsi conçue.

Pour englober ces deux configurations (axes concourants ou axes non concourants), on parlera ici plus généralement d'une liaison de type rotulaire.

L'invention n'est bien sûr pas limitée à ce qui vient d'être décrit, mais englobe toute variante entrant dans le cadre défini par les revendications.

En particulier, bien que des transmissions à câbles soit ici préférées car elles sont généralement plus compactes, plus fiables, et présentent une excellente régularité d'effort transmis, on pourra utiliser d'autres modes d'actionnement. Il reste néanmoins avantageux de prévoir un renvoi du mouvement du deuxième élément intermédiaire vers le support par un câble comme ici ou par un embiellage ou des arbres, de sorte que l'actionneur correspondant puisse être immobile et disposé sur le support 1.

## Revendications

1. Mécanisme d'épaule pour orthèse, assurant une articulation de type rotulaire entre un support (1) et un bras d'orthèse (100), comportant un premier élément intermédiaire (2) monté pour tourner sur le support autour d'un premier axe d'articulation (A1), un deuxième élément intermédiaire (4) monté pour tourner sur le premier élément intermédiaire selon un deuxième axe d'articulation (A2), le bras étant monté pour tourner sur le deuxième élément intermédiaire selon un troisième axe d'articulation (A3), le premier axe d'articulation (A1) s'étendant selon une direction horizontale longitudinale ; **caractérisé en ce que** le premier élément intermédiaire comporte un rail circulaire (3) sur lequel le deuxième élément intermédiaire (4) coulisse et qui présente un axe géométrique définissant le deuxième axe d'articulation (A2) qui s'étend verticalement lorsque le bras est au repos, le rail circulaire s'étendant pour faire extérieurement le tour de l'épaule.

2. Mécanisme d'épaule selon la revendication 1, dans lequel le mouvement du deuxième élément intermédiaire (4) le long du rail circulaire (3) du premier élément intermédiaire (2) est commandé par un actionneur (19) disposé sur le support 1.

3. Mécanisme d'épaule selon la revendication 2, dans lequel le deuxième élément intermédiaire (4) est attelé à un câble (10) qui est associé à des poulies (11,12,14,15) qui renvoient le câble vers une poulie menante (16) centrée sur l'axe d'articulation (A1) du premier élément intermédiaire (2) sur le support (1), la poulie menante (16) étant entraînée par l'actionneur disposé sur le support.

4. Mécanisme d'épaule selon la revendication 3, dans lequel l'actionneur (19) est un actionneur à câble qui agit sur un câble monté entre deux poulies d'axes parallèles (17,18) dont l'une est coaxiale à la poulie menante (16) et attelée à cette dernière.

5. Mécanisme d'épaule selon la revendication 1, dans lequel la rotation du premier élément intermédiaire (2) sur le support est commandé par un actionneur à câble (9) qui agit sur un câble monté entre deux poulies d'axes parallèles (7,8) dont l'une est coaxiale à l'axe A1 et attelée au premier élément intermédiaire (2).

6. Mécanisme d'épaule selon la revendication 1, dans lequel les premier, deuxième et troisième axes d'articulation sont sensiblement concourants.

7. Mécanisme d'épaule selon la revendication 1, dans lequel la somme d'une distance entre le premier et le deuxième axes d'articulation (A1,A2) et d'une distance entre le deuxième et le troisième axes d'articulation (A2,A3) est choisie pour être inférieure à un rayon (R_{humérus}) d'une sphère de dispersion d'une épaule appareillée au moyen du mécanisme d'épaule.

## Claims

1. A shoulder mechanism for an orthosis, providing a ball type joint between a support (1) and an orthosis upper arm (100), comprising a first intermediate element (2) mounted to pivot on the support about a first pivot axis (A1), a second intermediate element (4) mounted to pivot on the first intermediate element about a second pivot axis (A2), the upper arm being mounted to pivot on the second intermediate element about a third pivot axis (A3), the mechanism being **characterized in that** the first pivot axis (A1) extends in a longitudinal horizontal direction, the first intermediate element including a circular rail (3) on which the second intermediate element (4) slides and that presents a geometrical axis defining the second pivot axis (A2) that extends vertically when the upper arm is at rest, the circular rail extending to go around the shoulder on the outside.

2. A shoulder mechanism according to claim 1, wherein the movement of the second intermediate element (4) along the circular rail (3) of the first intermediate element (2) is controlled by an actuator (19) placed on the support (1).

3. A shoulder mechanism according to claim 2, wherein the second intermediate element (4) is coupled to a cable (10) that is associated with pulleys (11, 12, 14, 15) that deflect the cable towards a drive pulley (16) centered on the pivot axis (A1) of the first intermediate element (2) on the support (1), the drive pulley (16) being driven by the actuator placed on the support.

4. A shoulder mechanism according to claim 3, wherein the actuator (19) is a cable actuator that acts on a cable mounted between two pulleys (17, 18) of parallel axes,
one of them lying on the same axis as the drive pulley (16) and being coupled thereto.

5. A shoulder mechanism according to claim 1, wherein the pivoting of the first intermediate element (2) on the support is controlled by a cable actuator (9) that acts on a cable mounted between two axes (7, 8) of parallel axes, one of which coincides with the axis A1, and the corresponding pulley being coupled to the first intermediate element (2).

6. A shoulder mechanism according to claim 1, wherein the first, second, and third pivot axes are substantially concurrent.

7. A shoulder mechanism according to claim 1, wherein the sum of a distance between the first and second pivot axes (A1, A2) plus a distance between the second and third pivot axes (A2, A3) is selected to be less than a radius (Rₕᵤₘₑᵣᵤₛ) of a dispersion sphere of a shoulder fitted with the shoulder mechanism.

## Patentansprüche

1. Schultermechanismus für eine Orthese, der eine Gelenkverbindung der Art Kugelgelenk zwischen einem Träger (1) und einer Armorthese (100) sicherstellt, umfassend ein erstes Zwischenelement (2), das an dem Träger drehbar um eine erste Gelenkachse (A1) gelagert ist, ein zweites Zwischenelement (4), das an dem ersten Zwischenelement drehbar um eine zweite Gelenkachse (A2) gelagert ist, wobei der Arm an dem zweiten Zwischenelement drehbar um eine dritte Gelenkachse (A3) gelagert ist, wobei sich die erste Gelenkachse (A1) in einer horizontalen Längsrichtung erstreckt, **dadurch gekennzeichnet, dass** das erste Zwischenelement eine kreisförmige Schiene (3) umfasst, an der das zweite Zwischenelement (4) gleitet und die eine geometrische Achse aufweist, die die zweite Gelenkachse (A2) definiert, die sich vertikal erstreckt, wenn der Arm in Ruhestellung ist, wobei sich die kreisförmige Schiene so erstreckt, dass sie außen um die Schulter herumgeht.

2. Schultermechanismus nach Anspruch 1, wobei die Bewegung des zweiten Zwischenelements (4) entlang der kreisförmigen Schiene (3) des ersten Zwischenelements (2) von einem an dem Träger (1) angeordneten Aktuator (19) gesteuert wird.

3. Schultermechanismus nach Anspruch 2, wobei das zweite Zwischenelement (4) an einem Seil (10) angehängt ist, das mit Rollen (11, 12, 14, 15) verbunden ist, die das Seil zu einer Antriebsrolle (16) zurückführen, die auf die Gelenkachse (A1) des ersten Zwischenelements (2) an dem Träger (1) zentriert ist, wobei die Antriebsrolle (16) von dem an dem Träger angeordneten Aktuator angetrieben wird.

4. Schultermechanismus nach Anspruch 3, wobei der Aktuator (19) ein Aktuator mit Seil ist, der auf ein Seil einwirkt, das zwischen zwei Rollen (17, 18) mit parallelen Achsen gelagert ist, von denen eine koaxial zur Antriebsrolle (16) und an dieser letztgenannten angehängt ist.

5. Schultermechanismus nach Anspruch 1, wobei die Drehung des ersten Zwischenelements (2) an dem Träger von einem Aktuator (9) mit Seil gesteuert wird, der auf ein Seil einwirkt, das zwischen zwei Rollen (7, 8) mit parallelen Achsen gelagert ist, von denen eine koaxial zur Achse (A1) und an dem ersten Zwischenelement (2) angehängt ist.

6. Schultermechanismus nach Anspruch 1, wobei sich die erste, die zweite und die dritte Gelenkachse im Wesentlichen schneiden.

7. Schultermechanismus nach Anspruch 1, wobei die Summe eines Abstandes zwischen der ersten und der zweiten Gelenkachse (A1, A2) und eines Abstandes zwischen der zweiten und der dritten Gelenkachse (A2, A3) so gewählt wird, dass sie kleiner als ein Radius (R_{Oberarmknochen}) einer Dispersionskugel einer Schulter ist, die mittels eines Schultermechanismus nachgebildet wird.
